# EUROPÄISCHE PATENTSCHRIFT

(11) **EP 1 029 521 B1**
(45) Veröffentlichungstag und Bekanntmachung des Hinweises auf die Patenterteilung: **19.04.2006**
(21) Anmeldenummer: 00103308.3
(22) Anmeldetag: 18.02.2000
(51) Int. Cl.: A61F 13/04, A61F 13/00

(54) **Thermoplastisches Verbandmaterial und Verfahren zu dessen Herstellung**
Thermoplastic bandaging material and its manufacturing process
Pansement en matériau thermoplastique et son procédé de fabrication

(30) Priorität: 19.02.1999 DE 19907043
(43) Veröffentlichungstag der Anmeldung: 23.08.2000
(73) Patentinhaber: Karl Otto Braun KG, 67751 Wolfstein (DE)
(72) Erfinder: Langen, Günter, Dr., 67752 Wolfstein (DE); Meister, Marita, 67657 Kaiserslautern (DE); Burger, Joachim, Dr., 67657 Kaiserslautern (DE)
(74) Vertreter: HOFFMANN EITLE

(56) Entgegenhaltungen:
- EP-A- 0 305 842
- WO-A-94/03211
- WO-A-94/05339
- GB-A- 2 313 312
- US-A- 4 226 230
- US-A- 5 584 800
- US-A- 5 807 295

## Beschreibung

Die Erfindung betrifft ein thermoplastisches Verbandmaterial, insbesondere ein thermoplastisches Verbandmaterial in Rollenform, vorzugsweise für orthopädische und andere medizinische Anwendungen zum Ruhigstellen von Gliedmaßen und/oder Gelenken. Ferner betrifft die Erfindung ein Verfahren zur Herstellung dieses thermoplastischen Verbandmaterials.

Neben den seit langem verwendeten herkömmlichen Gipsverbandmaterialien sind seit einiger Zeit alternative Verbandmaterialien auf Kunststoffbasis bekannt. Diese besitzen gegenüber Gipsverbänden die Vorteile, daß sie verbesserte mechanische Eigenschaften besitzen, daß sie wasserunempfindlich und folglich abwaschbar sind, und aufgrund der schnellen Anlegbarkeit und Aushärtung und wegen ihres geringen Gewichts einen erhöhten Tragekomfort und eine verbesserte Mobilität gewährleisten. Darüber hinaus sind Verbandsysteme auf Kunststoffbasis im Gegensatz zu Gipsmaterialien für Röntgenstrahlen durchlässig und ermöglichen so Röntgennachuntersuchungen, ohne daß der Verband entfernt werden muß.

Sowohl die Gipsverbandmaterialien als auch die auf Kunststoff basierenden Verbandmaterialien sind im wesentlichen aus einem organischen oder anorganischen textilen Trägermaterial und dem darauf aufgebrachten Gips- bzw. Kunststoffmaterial zusammengesetzt. Bei den Kunststoffmaterialien ist zwischen irreversibel härtbaren Materialien und thermoplastischen, reversibel verformbaren Materialien zu unterscheiden.

Unter den irreversibel härtbaren Kunststoffverbandmaterialien haben sich in erster Linie wasserhärtende Systeme durchgesetzt, die als härtbare Kunststoffkomponenten reaktive Polyurethanprepolymere enthalten, die durch Kontakt mit Wasser aushärten. Durch geeignete Auswahl der Rezeptur können Verbandmaterialien erzielt werden, die nach Eintauchen in Wasser innerhalb einer Zeit aushärten, die das fachgerechte Anlegen und Modellieren des Verbandmaterials am Körper eines Menschen oder Tieres erlauben. Solange der Kunststoff nicht ausgehärtet ist, sind die einzelnen Lagen des Verbandmaterials miteinander verklebbar, wodurch letztendlich ein aus mehreren Schichten des Verbandmaterials bestehender Verband erhalten wird.

Bei thermoplastischen, reversibel verformbaren Verbandmaterialien wird die Selbstklebeeigenschaft durch Erwärmen des thermoplastischen Kunststoffes auf die jeweilige Erweichungstemperatur oder darüber erzielt. Beim Abkühlen erstarrt das Material wieder, wobei es auch bei Temperaturen unterhalb des Schmelzpunkts für einige Zeit plastisch und modellierbar bleibt. Nach dem Erstarren des thermoplastischen Harzes wird ein mehrlagig miteinander verbundenes Verbandsystem erhalten.

Obwohl derzeit auf dem Markt dominierend, sind die härtbaren Kunststoffverbandmaterialien, d.h. die wasserhärtbaren auf Polyurethanharzen basierenden Verbandmaterialien, gegenüber thermoplastischen Systemen nachteilhaft. Aufgrund der gewollten Eigenschaft der Wasserhärtbarkeit ist eine feuchtigkeitsfreie Produktion und eine aufwendige Verpackung des Produkts, die für Wasser und Luft undurchlässig ist, erforderlich. Darüber hinaus sind die wasserhärtbaren Harzrezepturen komplex und dementsprechend kostspielig. Auch bei Einhaltung möglichst feuchtigkeitsfreier Bedingungen ist die Lagerstabilität mit ca. 24 Monaten gering. Darüber hinaus sind die in Polyurethanharzen enthaltenen Isocyanate sensibilisierend, reizend und gesundheitsschädlich und daher insbesondere bei der Anwendung bei Risikopatienten nicht unbedenklich.

Ein sowohl den härtbaren als auch den thermoplastischen Kunststoffverbandmaterialien anhaftendes Problem ist, daß ein Verkleben der Lagen am Ort der Anwendung erwünscht und erforderlich ist, andererseits ein Verkleben der Lagen zu einem anderen Zeitpunkt als während der direkten Anwendung vermieden werden muß.

So beschreibt WO 95/19751 ein orthopädisches Verbandmaterial aus einem Trägergewebe und einem darauf aufgebrachten Material aus einem härtbaren Harz und einem darin gebundenen Füllstoff. Zur Verhinderung des Zusammenklebens der einzelnen Lagen des Verbandmaterials während der Lagerung ist dem Material ein flüchtiger wasserlöslicher Liner als Trennlage aufgebracht. Dieser Liner wird aufgelöst und entfernt, wenn das mit dem wasserhärtbaren Harz ausgerüstete Verbandmaterial zur Initiierung der Aushärtung mit Wasser in Kontakt gebracht wird.

Ein thermoplastisches Verbandmaterial ist in US 4 445 873 beschrieben. Es besteht aus einem flexiblen Stoffträger, typischerweise aus einem gestrickten Baumwollmaterial, und ist mit einem Polyesterharz beschichtet. Dieses beschichtete Material bleibt flexibel und kann in Rollenform gelagert werden. Damit die einzelnen Lagen des Materials beim Erweichen des Harzes in heißem Wasser nicht miteinander verkleben, bevor sie an der gewünschten Stelle auf den Körper aufgebracht werden, wird eine Polyethylenoxidschicht auf das Harz/Trägerkomposit aufgebracht. Diese Schicht löst sich beim Einweichen in Wasser auf und gibt so die selbstklebende Oberfläche des thermoplastischen Harzes frei.

In US 3 420 231 wird ein Verbandmaterial aus einem Träger und einem thermoplastischen Harz als bekannt beschrieben, das in aufgerollter Form vorliegt und zur Verhinderung des Verklebens benachbarter Lagen ein Trennmaterial aufweist, beispielsweise eine Papierzwischenlage. Diese Anordnung wird als unbefriedigend empfunden und US 3 420 231 schlägt vor, die vor der Anwendung zu entfernende Zwischenlage durch eine Beschichtung aus einem wasserlöslichen Harz mit inverser Löslichkeit in Wasser zu ersetzen. Durch die inverse Löslichkeit werden beim Erwärmen und Erweichen des thermoplastischen Harzes in heißem Wasser die Zwischenschichten nicht aufgelöst. Nach Anlegen des Verbandmaterials löst sich die Schutzschicht in dem anhaftenden, nunmehr abgekühlten Restwasser auf und ermöglicht ein Verkleben der einzelnen Lagen.

Schließlich offenbart US 4 143 655 ein orthopädisches Verbandmaterial, das aus einem textilen Träger und einem darauf aufgebrachten thermoplastischen Polymer aufgebaut ist. Zur Verhinderung des Verklebens benachbarter Lagen ist auf einer Seite des beschichteten Trägers als Trennlage ein Polyethylenfoliennetz aufgebracht. Dadurch wird beim Erwärmen des Verbandmaterials ein Verkleben der Lagen verhindert. Beim Anlegen des Verbandes wird die Polyethylenfolie abgezogen und so ein Verkleben der einzelnen Lagen miteinander ermöglicht.

Die oben geschilderten Maßnahmen zur Verhinderung des unerwünschten Verklebens härtbarer oder thermoplastischer Kunststoffverbandmaterialien bewirken eine gewisse Verbesserung gegenüber völlig ungeschützten Verbandmaterialsystemen, sind jedoch nach wie vor unbefriedigend und nicht geeignet, ein ungewünschtes Verkleben der Verbandmaterialien mit Sicherheit zu verhindern.

So geht bei Anwendung einer wasserlöslichen Schutzschicht die Schutzwirkung bereits im Wasserbad verloren, in der das Verbandmaterial auf die erforderliche Temperatur zum Erweichen des thermoplastischen Kunststoffes oder zur Initiierung des Aushärtens des wasserhärtbaren Kunststoffes eingebracht wird. Daher besteht die Möglichkeit, daß nach dem Entfernen aus dem Wasserbad oder bereits im Wasserbad ein Verkleben der benachbarten Lagen des zumeist in aufgerollter Form vorliegenden Verbandmaterials erfolgt.

Ferner ist der Einsatz eines wasserlöslichen oder invers wasserlöslichen Schutzfilmes mit der Beschränkung verbunden, daß zum Erwärmen des Materials ein Wasserbad verwendet werden muß. Eine Erwärmung durch beispielsweise ein Heißluftgebläse oder in einem Ofen ist nicht möglich.

Auch die Verwendung von vor der Anwendung zu entfernenden Zwischenlagen ist unbefriedigend, da die Handhabbarkeit durch die Materialtrennung im Moment der Anwendung deutlich erschwert wird und darüber hinaus unnötige Abfallmaterialien erzeugt werden.

WO 94/03211 offenbart ein thermoplastisches orthopädisches Verbandmaterial, das einen hochmolekulargewichtigen Polyester in Kombination mit einem Zellulosefüllstoff umfasst und bei einer Temperatur im Bereich von 55-70°C formbar und selbstklebend ist. Bei Bedarf kann eine Textilschicht in das Material inkorporiert oder daran angrenzend bereitgestellt sein.

US 5,807,295 beschreibt ein medizinisches Verbandmaterial, das zwei übereinander liegende Lagen aus beispielsweise einem gewebten, gestrickten oder Vliesmaterial umfasst, die durch Monofilamentstränge oder faserförmige Lagen voneinander beabstandet sind, und der Zwischenraum kann mit einem härtbaren Harz und/oder einem pharmakologischen Wirkstoff gefüllt werden.

WO 94/05339 betrifft Kompositmaterialien für z.B. orthopädische und physiotherapeutische Anwendungen, die durch Behandlung bei Temperaturen von nicht mehr als 90°C selbstklebende Eigenschaften entwickeln, und die in Form eines Laminats bei dieser Temperatur geformt werden können. Diese Kompositmaterialien umfassen einen ersten gummiartigen elastoviskosen Bestandteil mit einem Erweichungspunkt von nicht mehr als 90°C und einem zweiten semikristallinen Bestandteil, der im wesentlichen aus einem Polyester besteht und eine Schmelztemperatur von 35-80°C aufweist.

US 4,226,230 offenbart orthopädische Verbandmaterialien, die aus einem vernetzten Copolymer aus einem Laktonmonomer und einem polyfunktionalen Akrylatmonomer hergestellt werden. Dieses vernetzte Copolymer wird, gegebenenfalls in Kombination mit einem Trägersubstrat, über den Erweichungspunkt des Copolymers erwärmt, in diesem Zustand an den zu verbindenden Körperteil formangepasst und dann durch Abkühlen verfestigt. In einer Ausführungsform kann das erwärmte vernetzte Copolymer im erweichten Zustand selbstklebende Eigenschaften aufweisen, so dass übereinander zu liegen kommende Schichten miteinander verklebt werden.

Der vorliegenden Erfindung lag die Aufgabe zugrunde, ein thermoplastisches Verbandmaterial zu entwickeln, das bei Anlegen eines Verbandes gute Haftung der benachbarten Verbandmateriallagen ermöglicht, jedoch bis zu diesem Zeitpunkt ohne den Einsatz einer Trennlage ein Verkleben der Lagen effektiv verhindert, so daß das Material leicht abrollbar und leicht zu handhaben ist, und das nicht zur Erzeugung von Abfällen oder zur Kontaminierung des verwendeten Wasserbades führt.

Ferner soll ein Verfahren bereitgestellt werden, das die Herstellung eines solchen Verbandmaterials ein einer einfachen und kostengünstigen Weise ermöglicht.

Die obigen Aufgaben konnten erfindungsgemäß gelöst werden durch die Bereitstellung eines thermoplastischen Verbandmaterials, umfassend
i) eine erste dehnbare Textilbahn mit einem Flächengewicht im gedehnten Zustand von 50-300 g/m²,
ii) einen auf die erste Textilbahn aufgebrachten thermoplastischen Kunststoff mit einem Schmelzpunkt von 55-90°C, der bei Temperaturen von 50°C oder darunter starr oder restflexibel ist, aber nicht wesentlich erweicht wird, und im plastischen Zustand selbstklebend ist, und
iii) mindestens eine auf dieses Komposit aufgebrachte zweite dehnbare Textilbahn mit einem Flächengewicht im gedehnten Zustand von 10-80 g/m²,
wobei die erste und/oder mindestens eine zweite Textilbahn elastische Fäden oder Fasern enthält und das Verbandmaterial bei Temperaturen oberhalb des Schmelzpunkts des thermoplastischen Kunststoffs elastisch dehnbar ist.

Ferner konnten die Aufgaben erfindungsgemäß gelöst werden durch die Bereitstellung eines Verfahren zur Herstellung dieses thermoplastischen Verbandmaterials, das die folgenden Schritte umfaßt:
a) Aufbringen eines thermoplastischen Kunststoffs auf eine erste Textilbahn und
b) Aufbringen mindestens einer zweiten Textilbahn auf die mit dem thermoplastischen Kunststoff versehene erste Textilbahn aus Schritt a).

Bei der Herstellung oder spätestens beim Erwärmen des Verbandmaterials wird zwischen den oben genannten Bestandteilen eine haftende Verbindung untereinander hergestellt. Dadurch entsteht ein Komposit aus der mit dem thermoplastischen Kunststoff versehenen ersten Textilbahn und der darauf anhaftenden mindestens einen zweiten Textilbahn, das dann unter Verbleib aller Bestandteile in dem Verbandmaterial zu einem Verband verarbeitet werden kann.

Durch diese überraschende Lösung konnte ein neues thermoplastisches Verbandmaterial bereitgestellt werden, das nicht nur eine Nuancierung und Weiterentwicklung des Standes der Technik darstellt, sondern diesen durch die folgenden Vorteile erheblich erweitert:
- Die erwärmte Castbinde ist extrem leicht abrollbar, sogar nach kräftigem Ausdrücken des Restwassers nach Erwärmen im Wasserbad.
- Die zweite Textilbahn beeinflußt die Lagenhaftung im fertigen Verband nicht nachteilig.
- Die im fertigen Verband verbleibende zweite Textilbahn trägt gleichzeitig zu einer Erhöhung der Stabilität und zur Verbesserung der Luftdurchlässigkeit des Verbandes bei.
- Das Verbandmaterial kann nach dem Erwärmen eingesetzt werden, ohne daß eine Verklebungsschutzschicht entfernt werden muß. Dadurch ist die Handhabbarkeit beim Anlegen des Verbandes deutlich verbessert und es kommt nicht zur Kontamination des zum Erwärmen verwendeten Wasserbads.
- Die beim Anlegen des Verbandes nach außen gerichtete zweite Textilbahn kommt auf der Verbandoberfläche zu liegen, wodurch ein textiler Oberflächencharakter des Verbandes und dadurch ein erhöhter Tragekomfort bewirkt wird.

Nachfolgend werden das erfindungsgemäße thermoplastische Verbandmaterial sowie das Verfahren zu dessen Herstellung detaillierter beschrieben und anschließend anhand von konkreten Ausführungsbeispielen weiter illustriert.

Das erfindungsgemäße Verbandmaterial umfaßt mindestens eine erste dehnbare Textilbahn, einen auf die erste Textilbahn aufgebrachten thermoplastischen Kunststoff und eine auf dieses Komposit aufgebrachte zweite dehnbare Textilbahn.

Der hierin an verschiedenen Stellen verwendete Begriff "dehnbar" ist so zu verstehen, daß er dort, wo es technisch sinnvoll ist, unelastische und elastische Dehnbarkeit einschließt.

Die erste dehnbare Textilbahn ist eine textile Vliesbahn, ein Gelege oder eine gewirkte, gewebte oder gestrickte Textilbahn, vorzugsweise eine gewirkte erste Textilbahn, weiter bevorzugt ein gewirktes Band mit offenporiger Struktur. Vorzugsweise besitzt die erste Textilbahn elastisch dehnbare Eigenschaften.

Als Faser- bzw. Fadenmaterialien für die erste Textilbahn sind synthetische, regenerierte und natürliche Fasern sowie Mischungen daraus einsetzbar. Zur Erzielung der besonders vorteilhaften elastischen ersten Textilbahn werden textile Materialien aus Mischungen aus elastischen und unelastischen Fasern bzw. Fäden hergestellt.

Nichtelastische Fasern bzw. Fäden für die erste Textilbahn sind beispielsweise Baumwolle, Viskose sowie synthetische Fasern bzw. Fäden wie beispielsweise Polyacryl, Polyamid, Aramid, Polyester, Polyolefine oder anorganische Fasermaterialien, wie beispielsweise Glasfasern oder Kohlenstofffasern.

Elastische Faser- bzw. Fadenelemente sind beispielsweise Garne aus Elastodien, thermoplastischen Elastomeren, Elastan, elastische Polyamid- oder Polyurethanfasern, texturierte Synthesegarne, Zellulose-Zwirnkreppfäden oder Zellulose-Spinnkreppfäden.

Sowohl die elastischen als auch die nichtelastischen Fasern bzw. Fäden können in Abhängigkeit von der Stärke des gewünschten Trägermaterials bzw. Endprodukts in unterschiedlicher Stärke verwendet werden. Bei Vliesen liegt die Faserstärke bevorzugt bei 0,01-1 tex. Bei Gewirken, Gelegen, Gestricken und Geweben liegt die Fadenstärke der nichtelastischen Fäden normalerweise bei einer Fadenfeinheit von 4-200 tex (tex = Maßeinheit der Fadenfeinheit in g/1.000 m), vorzugsweise 10-110 tex, besonders bevorzugt 20-60 tex.

Die Fadenfeinheit der elastischen Fäden ist vorzugsweise etwas geringer und liegt bei 4-80 tex, vorzugsweise 10-40 tex, besonders bevorzugt 15-30 tex.

Die erste Textilbahn kann aus einer oder mehreren verschiedenen Arten von Fäden bzw. Fasern bestehen, die sich hinsichtlich des Materials und/oder der Garnstärke voneinander unterscheiden. Es können eine oder mehrere Arten nichtelastischer Garne und, soweit vorhanden, eine oder mehrere Arten elastischer Garne enthalten sein.

Besonders bevorzugt sind elastische erste Textilbahnen aus synthetischen Fäden bzw. Fasern aus einem oder mehreren synthetischen Materialien, z.B. Polyesterfasern oder Polyamidfasern, als nichtelastische Garne und Polyurethanfäden bzw. -Fasern als elastische Garne.

Wie bereits festgestellt, ist die erste Textilbahn unabhängig von ihrer textilen Herstellungsart (gewirkt, gewebt, gelegt, gestrickt oder ein Vlies) dehnbar und vorzugsweise elastisch sowohl in Längs- als auch in Querrichtung. Die Längsrichtung kennzeichnet hierbei die Bahnrichtung und die Querrichtung kennzeichnet die dazu senkrechte Richtung, die die Breite der ersten Textilbahn festlegt.

Die Längsdehnbarkeit beträgt vorzugweise von 30-200 %, weiter bevorzugt 60-110 %, besonders bevorzugt 85-100 %.

Die Querdehnbarkeit beträgt vorzugsweise 10-120 %, weiter bevorzugt 30-100 %, besonders bevorzugt 40-90 %.

Das Flächengewicht der ersten Textilbahn im gedehnten Zustand (in Bahnrichtung) beträgt 50-300 g/m², bevorzugt 70-200 g/m², besonders bevorzugt 80-120 g/m².

Die Definition des gedehnten Zustands erfolgt gemäß DIN 61632, Abschnitt 6.5. Folglich kennzeichnet die Fläche in "in Bahnrichtung gedehntem Zustand" die Fläche, die sich beim Anlegen einer Zugkraft von 10 N pro 1 cm Bindenbreite nach einer Kraftwirkungsdauer von 1 min. ergibt.

In der Regel ist die erste Textilbahn ungefärbt, d.h. zumeist weiß oder schwach farbig. Es besteht jedoch auch die Möglichkeit, eine gefärbte erste Textilbahn einzusetzen, die nach bekannten Textilfärbeverfahren erhalten werden kann.

Die Breite der ersten Textilbahn hängt von der gewünschten Breite des thermoplastischen Verbandmaterials ab. Übliche Maße für die Breite sind beispielsweise 5 cm, 7,5 cm, 10 cm oder 12,5 cm. Es sind jedoch ohne sonderliche Beschränkung schmalere oder breitere erste Textilbahnen einsetzbar.

Obwohl erste Textilbahnen unterschiedlicher Herstellungsart verwendet werden können, z.B. gewirkte, gelegte, gewebte, gestrickte oder Vlies-Materialien, sind gewirkte Textilbahnen besonders bevorzugt.

Die Größe der Porenöffnung der ersten Textilbahn ergibt sich aus der Fadenstärke der verwendeten Garnmaterialien und der Maschen- (bei gewirkten oder gestrickten Textilbahnen) oder Fadendichte (bei gewebten Textilbahnen) des Materials. Sie beträgt im ungedehnten Zustand ca. 0,5-100 mm², vorzugsweise 1-40 mm². Die Dicke der ersten Textilbahn im ungedehnten Zustand beträgt vorzugsweise 0,3-5 mm, weiter bevorzugt 1-2 mm.

Die oben als bevorzugt dargestellte Dehnbarkeit in Längs-und Querrichtung der ersten Textilbahn kann in dem Fachmann geläufiger Weise durch Auswahl der Verarbeitungsparameter bei der Herstellung der Textilbahn sowie durch eine geeignete Auswahl der Mengen, Dicken und Verhältnisse der in der Textilbahn enthaltenen Fasern eingestellt werden.

Der auf die erste Textilbahn aufgebrachte thermoplastische Kunststoff ist ein hydrolysestabiler lagerbeständiger Hotmelt-Klebstoff, der bei Temperaturen von 55-90°C, vorzugsweise 60-80°C, besonders bevorzugt 60-70°C schmilzt und auch nach Abkühlen unter den Schmelzpunkt für einige Zeit plastisch bleibt. Damit der thermoplastische Kunststoff in einem thermoplastischen Verbandmaterial unter den normalen Anwendungsbedingungen einsetzbar ist, muß er eine Temperaturbeständigkeit bis 50°C besitzen, vorzugsweise bis 55°C, d.h. es darf bei diesen Temperaturen nicht zu einer wesentlichen Erweichung oder zu einer Zersetzung des Kunststoffs kommen.

Vorzugsweise hat der thermoplastische Kunststoff einen Schmelzindex (125°C) von 0,5-200 g/10 Min., weiter bevorzugt 6-40 g/10 Min., besonders bevorzugt 12-25 g/10 Min., wobei die Bestimmung des Schmelzindex gemäß DIN ISO 1133 bei einer Prüftemperatur von 125 °C und einer Nominallast von 325 g erfolgt.

Die Aushärtezeit nach dem Erwärmen auf oder über den Schmelzpunkt hängt von der erreichten Temperatur und der Abkühlgeschwindigkeit ab und beträgt im allgemeinen 1-15 Minuten, vorzugsweise 2-10 Minuten, besonders bevorzugt 3-8 Minuten.

Geeignete thermoplastische Kunststoffe mit den oben genannten Eigenschaften sind beispielsweise Polyester, Polyurethan, Polyvinylacetat, oder auch die in der bereits erwähnten US 4 143 655 offenbarten Kunststoffe, vorzugsweise lineare gesättigte Polyesterverbindungen. Ein Beispiel für einen solchen Polyester ist das kommerziell erhältliche Polycaprolacton CAPA 640 (Hersteller: Solvay Interox, Warrington, GB)

Neben dem thermoplastischen Polymer kann der thermoplastische Kunststoff bei Bedarf weitere Hilfs- und Zusatzstoffe enthalten wie beispielsweise Farbpigmente, Stabilisatoren, Weichmacher, Harze, Tackyfier, UV-Filter, Füllstoffe, Antioxidationsmittel.

Es können auch Mischungen verschiedener Polymerverbindungen als thermoplastischer Kunststoff verwendet werden, soweit diese miteinander mischbar sind, und die erhaltene Mischung die oben beschriebenen Eigenschaften aufweist.

Ferner können auch thermoplastische Kunststoffe verwendet werden, die im erstarrten Zustand eine gewisse Restflexibilität aufweisen, anstatt vollkommen starr zu sein.

Beispiele hierfür sind Ethylen-Acrylsäureester-Copolymere, Ethyl-Vinylacetat-Copolymere und Polyurethane.

Durch den Einsatz solcher thermoplastischer Kunststoffe lassen sich Verbandmaterialien erhalten, die keine vollständige Immobilisierung des mit dem daraus hergestellten Verband versehenen Körperteils bewirkt, sondern eine lediglich semirigide Immobilisierung, die eine gesteuerte Bewegung und funktionelle Belastung der betroffenen Körperteile ermöglicht.

Die erfindungsgemäße, mindestens eine zweite dehnbare Textilbahn ist ein gewirktes, gewebtes oder gestricktes Material oder ein Vliesmaterial, vorzugsweise eine gewirkte Textilbahn.

Vorzugsweise ist die erfindungsgemäße zweite Textilbahn in Längs- als auch in Querrrichtung dehnbar, besonders bevorzugt elastisch. Die Längsrichtung kennzeichnet hierbei die Bahnrichtung und die Querrichtung kennzeichnet die dazu senkrechte Richtung, die die Breite der zweiten Textilbahn festlegt.

Das für die erfindungsgemäße zweite Textilbahn verwendete Faser- bzw. Fadenmaterial kann von beliebiger Natur sein, solange ein Verkleben von benachbarten Lagen des erfindungsgemäßen thermoplastischen Verbandmaterials verhindert wird, auch wenn der thermoplastische Kunststoff auf oder über seinen Schmelzpunkt hinaus erwärmt wird.

Als Faser- bzw. Fadenmaterialien für die zweite Textilbahn sind synthetische, regenerierte und natürliche Fasern bzw. Fäden sowie Mischungen daraus einsetzbar. Zur Erzielung der besonders vorteilhaften elastischen zweiten Textilbahn werden diese aus Mischungen aus elastischen und unelastischen Garnen hergestellt.

Als nichtelastische Fasern bzw. Fäden für die erfindungsgemäße zweite Textilbahn sind natürliche, synthetische sowie anorganische Materialien, wie beispielsweise Glasfasern oder Kohlenstofffasern unterschiedlicher Faser- bzw. Fadenstärke geeignet, vorzugsweise Baumwoll- oder Viskosefasern oder -Fäden. Diese können wahlweise in Kombination mit anderen synthetischen Fasern bzw. Fäden wie beispielsweise Polyacryl, Polyamid, Aramid, Polyester, Polyolefine, verwendet werden.

Elastische Fadenelemente für die bevorzugte elastische zweite Textilbahn sind beispielsweise Garne aus Elastodien, Elastan/Polyurethan, thermoplastischen Elastomeren, texturierten Synthesegarnen, Zellulose-Zwirnkreppfäden oder Zellulose-Spinnkreppfäden.

Bevorzugte Materialien sind elastische zweite Textilbahnen aus Viscose oder Baumwolle in Mischung mit elastischen Polyamid- oder Polyurethanfäden. Besonders bevorzugt sind elastische zweite Textilbahnen aus Viscose und Polyamid.

In einer erfindungsgemäß besonders bevorzugten Ausführungsform wird eine zweite Textilbahn eingesetzt, die aufgrund der enthaltenen Fasermaterialien und ihrer Herstellungsweise zwei Seiten mit unterschiedlicher Affinität zu (Kompatibilität mit) dem thermoplastischen Kunststoff aufweist. Eine solche zweite Textilbahn kann einerseits leicht mit der Seite, die eine hohe Affinität zu dem thermoplastischen Kunststoff aufweist, auf dem Komposit aus der ersten Textilbahn und dem thermoplastischen Kunststoff aufgebracht werden. Andererseits wird durch die dann nach außen zeigende Seite mit geringer Affinität eine besonders effektive Verhinderung des Verklebens von benachbarten Lagen des Verbandmaterials bewirkt.

Die unterschiedliche Affinität der zweiten Textilbahn zu dem thermoplastischen Kunststoff kann auch durch geeignete Beschichtungen einer oder beider Textilbahnseiten bewirkt werden. Mögliche Beschichtungsmittel hierbei sind beispielsweise Paraffine, Wachse, Oleophobierungsmittel oder Hydrophobierungsmittel.

Die vorzugsweise elastische erfindungsgemäße zweite Textilbahn besitzt eine Längsdehnbarkeit von vorzugsweise 30-200 %, weiter bevorzugt 60-160 %, besonders bevorzugt 80-130 %.

Die bevorzugte Querdehnbarkeit beträgt 10-120 %, weiter bevorzugt 30-100 %, besonders bevorzugt 40-90 %.

Die zweite Textilbahn kann die gleiche Breite besitzen wie die in dem thermoplastischen Verbandmaterial enthaltene erste Textilbahn, oder kann eine geringere Breite als die erste Textilbahn besitzen, sofern die verklebungsverhindernden Eigenschaften der zweiten Textilbahn dadurch nicht beeinträchtigt werden.

Das Flächengewicht der erfindungsgemäßen zweiten Textilbahn beträgt in gedehntem Zustand 10-80 g/m², bevorzugt 15-60 g/m², besonders bevorzugt 20-40 g/m², wobei der gedehnte Zustand so definiert ist, wie oben für die erste Textilbahn beschrieben.

Bevorzugt ist eine zweite Textilbahn, die ein Textilmaterial mit offenporiger Struktur darstellt, Besonders bevorzugt ein gewirktes Textilmaterial.

Die Größe der Porenöffnung der zweiten Textilbahn ergibt sich wie bei der ersten Textilbahn beschrieben aus der Faden- bzw. Faserstärke der verwendeten Materialien und der Maschen- oder Faden bzw. Faserdichte des Materials.

Die Porengröße in ungedehntem Zustand liegt bei etwa 0,1-100 mm², vorzugsweise 1-40 mm², weiter bevorzugt 1,5-10 mm².

Die Dicke der zweiten Textilbahn beträgt vorzugsweise 0,2-5 mm, weiter bevorzugt 0,5-2 mm, besonders bevorzugt 0,6-0,8 mm.

Das erfindungsgemäße thermoplastische Verbandmaterial umfaßt die erste Textilbahn, einen auf die erste Textilbahn aufgebrachten thermoplastischen Kunststoff und eine auf dieses Komposit aufgebrachte und darauf fest anhaftende zweite Textilbahn wie oben beschrieben.

Vorzugsweise wird der thermoplastische Kunststoff in aufgeschmolzener Form auf die erste Textilbahn aufgebracht, wodurch eine Beschichtung oder Imprägnierung erzielt wird. Diese Art des Aufbringens kann in dem Fachmann geläufiger Weise so durchgeführt werden, daß entweder die gesamte Faseroberfläche der ersten Textilbahn mit dem Kunststoff ummantelt oder eine offenporige Beschichtung erhalten wird.

Ferner ist es möglich, den thermoplastischen Kunststoff als feste Folie zwischen die erste und die zweite Textilbahn einzubringen, wodurch ein Verbandmaterial aus zunächst unverbundenen Schichten erhalten wird. Beim Erwärmen des Verbandmaterials schmilzt die Folie aus dem thermoplastischem Kunststoff auf und verbindet sich mit der ersten und der zweiten Textilbahn zu einem Kompositmaterial.

Ebenso ist es möglich, den Kunststoff nach einem Pulverstreuverfahren aufzubringen, bei dem der thermoplastische Kunststoff vorzugsweise als Pulver auf die erste Textilbahn aufgestreut und anschließend in einem Wärmekanal aufgeschmolzen wird.

Die auf der ersten Textilbahn aufgebrachte Menge an thermoplastischem Kunststoff ist variabel und kann zur Erzielung unterschiedlicher Festigkeiten des aus dem thermoplastischen Verbandmaterial herzustellenden Stützverbands eingestellt werden. Vorzugsweise beträgt die Auftragmenge in gedehntem Zustand der ersten Textilbahn 100-500 g/m², weiter bevorzugt 200-450 g/m², besonders bevorzugt 250-400 g/m², wobei der gedehnte Zustand wie oben definiert ist.

Der Anteil des thermoplastischen Kunststoffs in dem erfindungsgemäßen Verbandmaterial beträgt vorzugsweise 30-95 Gew.-%, weiter bevorzugt 50-90 Gew.-%, besonders bevorzugt 60-80 Gew.-%.

Das erfindungsgemäße thermoplastische Verbandmaterial ist oberhalb des Schmelzpunkts des thermoplastischen Kunststoffs vorzugsweise in Längs- und Querrichtung dehnbar. Die Dehnbarkeit wird durch die Eigenschaften der ersten Textilbahn und der zweiten Textilbahn beeinflußt und kann durch entsprechende Auswahl dieser Komponenten gesteuert und eingestellt werden.

Die Längsdehnbarkeit (wie oben definiert) beträgt vorzugsweise 30-120 %, weiter bevorzugt 50-110 %, besonders bevorzugt 60-100 %.

Die Querdehnbarkeit (wie oben definiert) beträgt vorzugsweise 20-120 %, weiter bevorzugt 30-100 %, besonders bevorzugt 40-90 %.

Das erfindungsgemäße thermoplastische Verbandmaterial liegt vorzugsweise in Rollenform vor, d.h. das bahnförmige Material wird zur Lagerung aufgerollt und verpackt, und kann vor der Verwendung in aufgerolltem Zustand erwärmt werden. Das erwärmte Material kann dann bequem zur Herstellung eines Verbands in geeigneter Geschwindigkeit abgewickelt und die einzelnen Lagen durch anpressen miteinander verklebt werden.

Das erfindungsgemäße Verfahren zur Herstellung des erfindungsgemäßen thermoplastischen Verbandmaterials umfaßt das Aufbringen des thermoplastischen Kunststoffes auf die erste Textilbahn und das anschließende Aufbringen der erfindungsgemäßen mindestens einen zweiten Textilbahn auf die mit dem thermoplastischen Kunststoff versehene erste Textilbahn.

Dabei wird in einer Ausführungsform zunächst die erste Textilbahn, soweit es sich um eine dehnbare erste Textilbahn handelt, in gedehntem Zustand bei einer Temperatur mit dem thermoplastischen Kunststoff beschichtet, bei der der thermoplastische Kunststoff in geschmolzener Form auf das Trägermaterial aufgebracht werden kann.

Der anwendbare Temperaturbereich liegt zwischen der Schmelztemperatur des verwendeten thermoplastischen Kunststoffes und einer Temperatur, bei der der thermoplastische Kunststoff nicht zu dünnflüssig ist und keine Zersetzung auftritt. Bevorzugt sind Temperaturen im Bereich von 70 bis 150°C, weiter bevorzugt 80 bis 120°C, besonders bevorzugt 90 bis 110°C.

Der oben beschriebene Imprägnier- oder Beschichtungsschritt kann mit herkömmlichen Vorrichtungen durchgeführt werden, die üblicherweise zur Imprägnierung von textilen Materialien mit Kunststoffen verwendet werden.

Anschließend wird die erfindungsgemäße mindestens eine zweite Textilbahn auf das Komposit aus dem thermoplastischen Kunststoff und der ersten Textilbahn aufgebracht. Vorzugsweise geschieht dies bei einer Temperatur, die bei der Schmelztemperatur des thermoplastischen Kunststoffes oder darüber liegt, so daß die zweite Textilbahn durch leichtes Andrücken teilweise in den Kunststoff hineingedrückt und dadurch nach dessen Erstarrung darauf festgehalten wird.

Daher kann die zweite Textilbahn direkt im Anschluß an den Imprägnier- oder Beschichtungsschritt aufgebracht werden, bevor der thermoplastische Kunststoff vollständig erstarrt ist. Es ist jedoch auch möglich, das bereits abgekühlte imprägnierte Material erneut auf eine Temperatur oberhalb des Schmelzpunktes des thermoplastischen Kunststoffes zu erwärmen und dann die zweite Textilbahn aufzubringen.

Es ist ferner möglich, die zweite Textilbahn bei Raumtemperatur auf das Komposit aus dem thermoplastischen Kunststoff und der ersten Textilbahn aufzulegen. Die Verbindung miteinander erfolgt dannn beim Schmelzen des Kunststoffs bei der Anwendung des Verbandmaterials.

Alternativ dazu kann die erfindungsgemäße zweite Textilbahn mit einem geeigneten Klebstoff auf das Komposit aus dem thermoplastischen Kunststoff und der ersten Textilbahn aufgebracht werden. Dabei ist der Klebstoff so auszuwählen, daß keine Ablösung der zweiten Textilbahn von dem Komposit erfolgt, wenn das erfindungsgemäße thermoplastische Verbandmaterial vor der Anwendung erwärmt wird, und daß der Klebstoff keine nachteiligen Auswirkungen auf die Eigenschaften der zweiten Textilbahn zeigt. Geeignete Klebstoffe zur Befestigung des Trennlagenmaterials auf dem imprägnierten Trägermaterial sind zum Beispiel Hot-melt Klebstoffe, deren Schmelztemperatur oberhalb der Temperaturen liegt, auf die das Verbandmaterial bei der Anwendung erwärmt wird.

In einer weiteren Ausführungsform des erfindungsgemäßen Verfahrens werden die erste Textilbahn, eine Folie aus dem thermoplastischen Kunststoff und die mindestens eine zweite Textilbahn in dieser Anordnung übereinandergelegt und zu einer geeigneten Lagerform verarbeitet, vorzugsweise zu einem Verbandmaterial in Rollenform.

Das erfindungsgemäße thermoplastische Verbandmaterial ist vielseitig einsetzbar zur Herstellung von Verbänden zur Stützung oder Ruhigstellung von Körperteilen von Menschen oder Tieren. Es ist einfach und kostengünstig herzustellen und nahezu unbegrenzt lagerstabil, ohne daß eine hermetisch Dichte Verpackung notwendig ist.

Bei der Anwendung bleibt der verklebungsverhindernde Effekt des erfindungsgemäßen Verbandmaterials bis unmittelbar zu dem Zeitpunkt des Aufbringens auf den mit dem Verband zu versehenden Körperteil erhalten. Dadurch ist auch bei kräftigem Ausdrücken des Restwassers nach dem Erwärmen eine sehr gute Abrollbarkeit des Verbandmaterials gewährleistet. Andererseits bewirkt die im Verband verbleibende zweite Textilbahn keine nachteilige Beeinträchtigung der Anhaftung benachbarter Verbandmateriallagen im angelegten Verband und führt gleichzeitig zu einer erhöhten Stabilität wie auch zu einer Verbesserung der Luftdurchlässigkeit des resultierenden Verbandes.

Da die zweite Textilbahn im Verbandmaterial verbleibt, ist das Material sehr leicht zu handhaben. Ferner erhält die Außenseite des resultierenden Verbandes durch die beim Anlegen des Verbandes nach außen gerichtete zweite Textilbahn einen textilen Charakter, wodurch der Tragekomfort im Vergleich zu bisher bekannten Verbandmaterialien auf Kunststoffbasis erheblich erhöht wird.

Nachfolgend wird die vorliegende Erfindung anhand von konkreten Ausführungsbeispielen weiter erläutert.

In den Beispielen kennzeichnen die Bezeichnungen "L1" bis "L4" die Legeschienen auf der Wirkmaschine und "f" kennzeichnet die Anzahl der Filamente einer Faser oder eines Fadens.

### Beispiel 1

### 1. erste Textilbahn

Auf einer Bandwirkmaschine (Hersteller: Comez SpA, Cilavegna PV, Italien) mit 3 Legeschienen wurde eine gewirkte Textilbahn mit offenporiger Struktur hergestellt.
Erhaltne Garne:
L1: 16,7 tex f48x1 Polyester texturiert (Masche) - elastisches Garn
L2: 55 tex f96x1 Polyester (Teilschluß) - Multifilamentgarn
L3: 8 tex Polyurethan/Elasthan (Dorlastan - Hersteller: GVW Veredelungswerke GmbH, Goch, Deutschland) umflort mit 7,8 tex f34x1 Polyamid (Teilschluß) - elastisches Garn

Endtex gedehnt: 10,2
(Endtex = Feinheit des Garnes bei maximalem Verzug)

| Bindung: | | Fadenzahl: |
|---|---|---|
| L1 | 2.1 - 2.1 (Masche) | 61 |
| L2 | 1.4 - 4.1 | 59 |
| L3 | 1.2 - 2.1 | 61 |

Teilung: E15
Einzug: voll

Die Fadenschar der L3 wurde der Wirkmaschine mit einer Federspannung von 10-13 cN zugeführt. In diesem Zustand werden 40 Maschenreihen/10cm (gedehnt - DIN 61632) eingearbeitet. Das aus den obigen Garnen gewirkte Material weist eine Fertigbreite von 10 cm (ungedehnt) auf. Unter Anwendung der Meßmethode der DIN 61632 wurde ein Flächengewicht von 108 g/m² (gedehnt, bei einer Belastung mit 10 N/cm) bestimmt. Die Dehnbarkeit in Längsrichtung betrug 45 %, in Querrichtung 85 %.

### 2. Thermoplastischer Kunststoff

Eingesetzt wurde das kommerziell erhältliche Handelsprodukt CAPA 640 (Hersteller: Solvay Interox, Warrington, GB), ein Polycaprolacton in Granulatform mit einem Schmelzpunkt von 57°C.

### 3. Zweite Textilbahn

Auf einer weiteren Bandwirkmaschine (Hersteller: Comex SpA, Cilavegna PV, Italien) mit zwei Legeschienen wurde eine gewirkte Textilbahn mit offener Netzstruktur hergestellt.

### Erhaltne Garne:

L1: 8 tex f17x1 Polyamid texturiert (Masche)

L2: 17 tex Viscose (Teilschluß)

| Bindung: | Fadenzahl: | Einzug: |
|---|---|---|
| L1 2.1-2.1 (Masche) | 31 | 1 voll - 1 leer |
| L2 1.9-9.1 | 21 | 1 voll - 1 leer - |
| | | 1 voll - 3 leer |

### Teilung: E15

Die oben beschriebenen Garne werden mit der Einstellung 30 Maschen/10 cm (gedehnt - DIN 61632) verarbeitet. Durch eine Behandlung des Materials mit Wasserdampf schrumpft die Textilbahn in Längsrichtung, wobei eine Längsdehnbarkeit von 150 % (DIN 61632) gemessen wurde, die Breite der Textilbahn betrug 9 cm. Unter Anwendung der Meßmethode der DIN 61632 wurde ein Flächengewicht von 17 g/m² (gedehnt, bei einer Belastung von 10 N/cm) bestimmt. Die Dehnbarkeit in Querrichtung betrug 35 %.

Der thermoplastische Kunststoff wurde durch einen Doppelschnecken-Extruder (LD = 15/50) mit nachgeschalteter, beheizter Schlitzdüse bei einer Temperatur von 120°C aufgeschmolzen und unter Erhalt der Offenporigkeit und der Längs-/Querdehnbarkeit auf die erste Textilbahn aufgebracht. Auftragsmenge 360 g/m² (gedehnt - DIN 61632) Die zweite Textilbahn wurde unter Erhalt der Dehnung unmittelbar auf die mit dem thermoplastischen Kunststoff beschichtete erste Textilbahn aufkaschiert. Das erhaltene thermoplastische Verbandmaterial wurde nach Abkühlen auf Raumtemperatur in 2,80 m lange Stücke geschnitten und zu Binden gewickelt..

Zur Anwendung wurden die Binden in einem Wasserbad bei 70°C ca. 5 Minuten erwärmt und in gewohnter Weise am Patienten angewendet. Der therapeutische Nutzen des Verbandmaterials ist vergleichbar mit dem kommerziell erhältlichen Produkt Articast S (Hersteller: Beiersdorf AG, Hamburg, Deutschland), eine Castbinde auf Basis feuchtigkeitshärtender Polyurethan-Systeme.

### Beispiel 2

### 1. erste Textilbahn

Auf einer Bandwirkmaschine (Hersteller: Müller, Frick, Schweiz, Typ Raschelina) mit vier Legeschienen wurde eine gewirkte Textilbahn mit offener Netzstruktur mit einem Flächengewicht im gedehnten Zustand von 100 g/m², einer Längsdehnbarkeit von 85 % und einer Querdehnbarkeit von 60 % hergestellt.

Enthaltene Garne:
L1: 28 tex f48x1 Polyester (Masche)
L2: 55 tex f96x1 Polyester (Teilschuß)
L3: 16 tex Polyurethan mit 11 tex f34x1 Polyamid (Teilschuß) - elastisches Garn, Reißdehnnung: 277 %
L4: 55 tex f96x1 Polyester (Teilschuß)

Bindung: L1: 2.0 - 0.2
L2: 0.6 - 6.0
L3: 0.1 - 1.0
L4: 6.1 - 1.6

Teilung: E 18

Einzug: L1, L2, L3, L4: 1 voll - 1 leer

Die Fadenschar der L3 wurde der Wirkmaschine unter einem Verzug von 130 % zugeführt.

Das aus den obigen Garnen gewirkte Material wies eine Maschenreihenzahl von 24/10 cm (gedehnt) sowie 34 Maschenstäbe/10 cm (ungedehnt) auf. Die Breite der ersten Textilbahn betrug ungedehnt 10 cm.

### 2. Thermoplastischer Kunststoff

Eingesetzt wurde das kommerziell erhältliche Handelsprodukt CAPA 640 (Hersteller: Solvay Interox, Warrington, GB), ein Polycaprolacton in Pulverform mit einem Schmelzpunkt von 57°C.

### 3. zweite Textilbahn

Auf einer weiteren Bandwirkmaschine mit drei Legeschienen wurde eine gewirkte zweite Textilbahn mit offener Netzstruktur mit einem Flächengewicht im gedehnten Zustand von 25 g/m², einer Längsdehnbarkeit von 120 % und einer Querdehnbarkeit von 50 % hergestellt.

Enthaltene Garne:
L1: 4,4 tex f13x1 Polyamid texturiert (Masche) - elastisches Garn.
L2: 17 tex Viscose (Teilschuß)
L3: 17 tex Viscose (Teilschuß)

Bindung: L1: 2.0 - 0.2
L2: 2.2 - 0.0 - 0.0 - 0.0 - 2.2 - 2.2
L3: 0.0 - 4.4 - 8.8 - 12.12 - 8.8 - 4.4

Teilung: E 14

Einzug: L1, L2: voll
L3: 1 voll - 1 leer - 1 voll - 2 leer

Die aus den oben beschriebenen Garnen gewirkte zweite Textilbahn wies 60 Maschenreihen/10 cm (gedehnt) und 56 Maschenstäbe/10 cm (ungedehnt) auf. Die Dehnbarkeit wurde durch eine Behandlung des Materials mit Wasserdampf erzielt. Die Breite der zweiten Textilbahn betrug ungedehnt 10 cm.

Der thermoplastische Kunststoff wurde mit einem Hotmelt-Auftragsaggregat, bestehend aus Schmelztank und Schlitzdüse, mit einer Temperatur von 100 °C auf die erste Textilbahn unter Erhalt der Offenporigkeit und der Längs-/Querdehnbarkeit aufgebracht. Auftragsmenge: 310 g/m² (gedehnt). Die zweite Textilbahn wurde unter Erhalt der Dehnung unmittelbar auf das Komposit aus thermoplastischem Kunststoff und der ersten Textilbahn aufkaschiert. Das erhaltene thermoplastische Verbandmaterial wurde nach Abkühlen auf Raumtemperatur in 2,50 m lange Stücke geschnitten und zu Binden gewickelt.

Zur Anwendung wurden die Binden in einem Wasserbad bei 70 °C 5 Minuten erwärmt und zu einem 3-lagigen Formkörper gewickelt. Die Lagenhaftung sowie die erreichte Festigkeit sind vergleichbar mit dem kommerziell erhältlichen Produkt Articast S (Hersteller: Beiersdorf AG, Hamburg, Deutschland), eine Castbinde auf Basis feuchtigkeitshärtender Polyurethan-Systeme

### Beispiel 3

### 1. erste Textilbahn

Auf einer Bandwirkmaschine (Hersteller: Müller, Frick, Schweiz, Typ Raschelina) mit zwei Legeschienen wurde eine gewirkte Textilbahn mit offener Netzstruktur mit einem Flächengewicht im gedehnten Zustand von 93 g/m² sowie einer Längsdehnbarkeit von 60 % und einer Querdehnbarkeit von 80 % hergestellt.

Enthaltene Garne:
L1: 16,7 tex f30x1 Polyester texturiert (Masche) - elastisches Garn.
L2: 55 tex f96x1 Polyester (Teilschuß)
Bindung:
   L1: 2.0 - 0.2
   L2: 0.0 - 6.6
Teilung: E 9
Einzug:
   L1, L2: voll

Das aus den obigen Garnen gewirkte Material wies eine Maschenreihenzahl von 40/10 cm (gedehnt) sowie 43 Maschenstäbe/10 cm auf (ungedehnt) auf. Die Dehnbarkeit erhielt das Material durch eine Wasserdampfbehandlung. Die Breite der ersten Textilbahn betrug 10 cm.

### 2. Thermoplastischer Kunststoff

Eingesestzt wurde das kommerziell erhältliche Produkt Unex 4103 (Hersteller: Dakota Coatings, Neerhonderd, Belgien), ein Polyurethan mit einem Schmelzbereich von 60 - 70 °C und einer Temperaturbeständigkeit bis 50°C.

### 3. zweite Textilbahn

Eingesetzt wurde das kommerziell erhältliche Handelsprodukt Co-Flex (Hersteller: Andover Ltd., Salisbury MA, USA), eine kohäsive Polyamid Vliesbinde mit feinporiger Struktur, einem Flächengewicht im gedehnten Zustand von 52 g/m², einer Längsdehnbarkeit von 160 % und einer Querdehnbarkeit von 25 %.

### Bestandteile der Vliesbinde:

Das Vlies-Grundmaterial der zweiten Textilbahn bestand aus Polyamid mit 44 Polyurethanfäden (Fadenstärke 8,8 tex)/10 cm als elastischem Bestandteil.

Auf die gedehnte erste Textilbahn mit einem Flächengewicht von 93 g/m² wurde der thermoplastische Kunststoff als Pulver in einer Menge von 310 g/m² aufgestreut und in einem Warmluftdurchlaufofen aufgeschmolzen. Anschließend wurde die zweite Textilbahn auf das Komposit aus thermoplastischem Kunststoff und der ersten Textilbahn aufkaschiert. Das erhaltene thermoplastische Verbandmaterial mit einer Breite von 10 cm wies eine Längsdehnbarkeit von 60 % und eine Querdehnbarkeit von 70 % auf.

## Patentansprüche

1. Thermoplastisches Verbandmaterial, umfassend
i) eine erste dehnbare Textilbahn mit einem Flächengewicht im gedehnten Zustand von 50-300 g/m²,
ii) einen auf die erste Textilbahn aufgebrachten thermoplastischen Kunststoff mit einem Schmelzpunkt von 55-90°C, der bei Temperaturen von 50°C oder darunter starr oder restflexibel ist, aber nicht wesentlich erweicht wird, und im plastischen Zustand selbstklebend ist, und
iii) mindestens eine auf dieses Komposit aufgebrachte zweite dehnbare Textilbahn mit einem Flächengewicht im gedehnten Zustand von 10-80 g/m²,
wobei die erste und/oder mindestens eine zweite Textilbahn elastische Fäden oder Fasern enthält und das Verbandmaterial bei Temperaturen oberhalb des Schmelzpunkts des thermoplastischen Kunststoffs elastisch dehnbar ist.

2. Verbandmaterial gemäß Anspruch 1, **dadurch gekennzeichnet, daß** die Längsdehnbarkeit des Verbandmaterials bei einer Temperatur oberhalb des Schmelzpunkts des thermoplastischen Kunststoffs mindestens 30 % und die Querdehnbarkeit mindestens 10 % beträgt.

3. Verbandmaterial gemäß mindestens einem der vorhergehenden Ansprüche, **dadurch gekennzeichnet, daß** die erste und/oder die mindestens eine zweite Textilbahn ein Gewirke, Gestricke, Gewebe, Gelege oder Vlies ist.

4. Verbandmaterial gemäß mindestens einem der vorhergehenden Ansprüche, **dadurch gekennzeichnet, daß** die erste Textilbahn ein Flächengewicht im gedehnten Zustand von 50-200 g/m² und die zweite Textilbahn ein Flächengewicht im gedehnten Zustand von 10-50 g/m² aufweist.

5. Verbandmaterial gemäß mindestens einem der vorhergehenden Ansprüche, **dadurch gekennzeichnet, daß** der Anteil des thermoplastischen Kunststoffs in dem Verbandmaterial 30-95 Gew.-% beträgt.

6. Verbandmaterial gemäß mindestens einem der vorhergehenden Ansprüche, **dadurch gekennzeichnet, daß** der thermoplastische Kunststoff einen Schmelzindex (125°C) von 0,5-200 g/10 Min. aufweist.

7. Verbandmaterial gemäß mindestens einem der vorhergehenden Ansprüche, **dadurch gekennzeichnet, daß** der nicht elastische Anteil der ersten Textilbahn Fasern und/oder Fäden aus Zellulose, Baumwolle, Viscose, Polyacryl, Polyamid, Aramid, Polyester, Polyolefinen, Glas oder Kohlenstoff oder Mischungen daraus umfaßt.

8. Verbandmaterial gemäß mindestens einem der vorhergehenden Ansprüche, **dadurch gekennzeichnet, daß** der nicht elastische Anteil der zweiten Textilbahn Fasern oder Fäden aus Zellulose, Baumwolle, Glas oder Kohlenstoff, wahlweise in Kombination mit anderen synthetischen Fasern bzw. Fäden aus Polyacryl, Polyamid, Aramid, Polyester oder Polyolefinen oder Mischungen daraus umfaßt.

9. Verbandmaterial gemäß mindestens einem der vorhergehenden Ansprüche, **dadurch gekennzeichnet, daß** der elastische Anteil der ersten und zweiten Textilbahn Fasern oder Fäden aus Elastodien, thermoplastischen Elastomeren, Elastan, texturierten Synthesegarnen, Zellulose-Zwirnkreppfäden oder Zellulose-Spinnkreppfäden oder Mischungen daraus umfaßt.

10. Verbandmaterial gemäß mindestens einem der vorhergehenden Ansprüche, **dadurch gekennzeichnet, daß** die zweite Textilbahn zwei Seiten mit unterschiedlicher Affinität zu oder Kompatibilität mit dem thermoplastischen Kunststoff aufweist.

11. Verfahren zur Herstellung eines thermoplastischen Verbandmaterials gemäß mindestens einem der Ansprüche 1 bis 10, **dadurch gekennzeichnet, daß** es die folgenden Schritte umfaßt:
a) Aufbringen eines thermoplastischen Kunststoffs auf eine erste Textilbahn und
b) Aufbringen mindestens einer zweiten Textilbahn auf die mit dem thermoplastischen Kunststoff versehene erste Textilbahn aus Schritt a).

12. Verfahren gemäß Anspruch 11, **dadurch gekennzeichnet, daß** es ferner den folgenden Schritt umfaßt:
c) Aufwickeln des aus Schritt b) erhaltenen Materials zu einem rollenförmigen Verbandmaterial.

## Claims

1. Thermoplastic bandaging material, including
i) a first extendible textile web with a weight per unit area in the extended state of 50-300 g/m²,
ii) a thermoplastic polymer applied to the first textile web and having a melting point of 55-90°C, which at temperatures of 50°C or below is rigid or has a residual flexibility but is not substantially softened, and in the plastic state is self-adhesive, and
iii) at least one second extendible textile web applied to this composite with a weight per unit area in the extended state of 10-80 g/m²,
wherein the first and/or at least one second textile web contains elastic threads or fibres and the bandaging material is elastically extendible at temperatures above the melting point of the thermoplastic polymer.

2. Bandaging material according to claim 1, **characterised in that** the longitudinal extendibility of the bandaging material at a temperature above the melting point of the thermoplastic polymer is at least 30% and the transverse extendibility is at least 10%.

3. Bandaging material according to one or more of the preceding claims, **characterised in that** the first and/or the at least one second textile web is a knitted, interlaid, woven or non-woven fabric.

4. Bandaging material according to one or more of the preceding claims, **characterised in that** the first textile web has a weight per unit area in the extended state of 50-200 g/m² and the second textile web has a weight per unit area in the extended state of 10-50 g/m².

5. Bandaging material according to one or more of the preceding claims, **characterised in that** the proportion of thermoplastic polymer in the bandaging material is 30-95 wt.%.

6. Bandaging material according to one or more of the preceding claims, **characterised in that** the thermoplastic polymer has a melt flow index (125°C) of 0.5-200 g/10 min.

7. Bandaging material according to one or more of the preceding claims, **characterised in that** the non-elastic fraction of the first textile web includes fibres and/or threads of cellulose, cotton, viscose, polyacryl, polyamide, aramid, polyester, polyolefins, glass or carbon or mixtures thereof.

8. Bandaging material according to one or more of the preceding claims, **characterised in that** the non-elastic fraction of the second textile web includes fibres or threads of cellulose, cotton, glass or carbon, optionally in combination with other synthetic fibres or threads of polyacryl, polyamide, aramid, polyester or polyolefins or mixtures thereof.

9. Bandaging material according to one or more of the preceding claims, **characterised in that** the elastic fraction of the first and second textile webs includes fibres or threads of elastodiene, thermoplastic elastomers, elastane, textured synthetic yarns, cellulose twisted crêpe threads or cellulose spun crêpe threads or mixtures thereof.

10. Bandaging material according to one or more of the preceding claims, **characterised in that** the second textile web has two sides with different affinity for or compatibility with the thermoplastic polymer.

11. Method for the manufacture of a thermoplastic bandaging material according to one or more of claims 1 to 10, **characterised in that** it includes the following steps:
a) applying a thermoplastic polymer to a first textile web and
b) applying at least one second textile web to the first textile web provided with the thermoplastic polymer from step a).

12. Method according to claim 11, **characterised in that** it further includes the following step:
c) winding up the material obtained from step b) into a roll-like bandaging material.

## Revendications

1. Pansement en matériau thermoplastique, comprenant :
i) une première bande textile extensible, ayant, à l'état étiré, un poids surfacique de 50 à 300 g/m²,
ii) un matériau synthétique thermoplastique, appliqué sur la première bande textile, ayant un point de fusion de 55 à 90°C, rigide ou présentant une flexibilité résiduelle à des températures de 50°C ou moins, mais ne s'amollissant pratiquement pas et étant auto-adhésif à l'état plastique, et
iii) au moins une deuxième bande textile extensible, appliquée sur ce composite, ayant à l'état étiré un poids surfacique de 10 à 80 g/m²,
la première et/ou au moins une deuxième bande textile contenant des fils ou des fibres élastiques, et le pansement étant extensible élastiquement à des températures supérieures au point de fusion du matériau synthétique thermoplastique.

2. Pansement selon la revendication 1, **caractérisé en ce que** l'extensibilité longitudinale du pansement, à une température supérieure au point de fusion du matériau synthétique thermoplastique, est d'au moins 30 % et l'extensibilité transversale est d'au moins 10 %.

3. Pansement selon au moins l'une des revendications précédentes, **caractérisé en ce que** la première et/ou la au moins une deuxième bande textile est un tricot, un produit tricoté, un tissu, un produit garni ou un voile de fibres.

4. Pansement selon au moins l'une des revendications précédentes, **caractérisé en ce que** la première bande textile présente à l'état étiré un poids surfacique de 50 à 200 g/m², et la deuxième bande textile présente à l'état étiré un poids surfacique de 10 à 50 g/m².

5. Pansement selon au moins l'une des revendications précédentes, **caractérisé en ce que** la proportion de matière synthétique thermoplastique dans le pansement est de 30 à 95 % en poids.

6. Pansement selon au moins l'une des revendications précédentes, **caractérisé en ce** le matériau synthétique thermoplastique présente un indice de fusion (à 125°C) de 0,5 à 200 g/ 10 min.

7. Pansement selon au moins l'une des revendications précédentes, **caractérisé en ce que** la proportion non élastique de la première bande textile comprend des fibres et/ou des fils en cellulose, coton, viscose, polyacryl, polyamide, aramide, polyester, polyoléfines, verre ou carbone, ou des mélanges de ceux-ci.

8. Pansement selon au moins l'une des revendications précédentes, **caractérisé en ce que** la proportion non élastique de la deuxième bande textile comprend des fibres et/ou des fils en cellulose, coton, verre ou carbone, au choix en combinaison avec d'autres fibres ou fils synthétiques en polyacryl, polyamide, aramide, polyester, ou des polyoléfines, ou des mélanges de ceux-ci.

9. Pansement selon au moins l'une des revendications précédentes, **caractérisé en ce que** la proportion élastique de la première et de la deuxième bande textile comprend des fibres et/ou des fils en élastodiène, en élastomère thermoplastique, en élasthanne, en fil de synthèse texturé, en fil de crêpe torsadée en cellulose, ou en fil de crêpe filé en cellulose ou des mélanges de ceux-ci.

10. Pansement selon au moins l'une des revendications précédentes, **caractérisé en ce que** la deuxième bande textile présente deux faces à affinité envers le matériau synthétique, ou à compatibilité avec le matériau synthétique différente.

11. Procédé de fabrication d'un pansement en matériau thermoplastique selon au moins l'une des revendications 1 à 10, **caractérisé en ce qu'**il comprend les étapes suivantes :
a) application d'un matériau synthétique thermoplastique sur une première bande textile, et
b) application d'au moins une deuxième bande textile sur la première bande textile, issue de l'étape a), munie de la matière synthétique thermoplastique.

12. Procédé selon la revendication 11, **caractérisé en ce qu'**il comprend en outre l'étape suivant :
c) enroulement du matériau de pansement obtenu à l'étape b), pour donner un pansement en forme de rouleau.
